# EUROPEAN PATENT APPLICATION

(11) **EP 0 525 393 A1**
(43) Date of publication of application: **03.02.1993**
(21) Application number: 92110809.8
(22) Date of filing: 26.06.1992
(51) Int. Cl.: A61F 5/37

(54) **Limb-restraining safety harness**

(30) Priority: 28.06.1991 US 723049
(71) Applicant: MCCARTHY, Andrew D., Bethesda, MD 20816 (US)
(72) Inventor: MCCARTHY, Andrew D., Bethesda, MD 20816 (US)
(74) Representative: VOSSIUS & PARTNER

(57) **Abstract**

An associated safety harness about the lower torso of a seated patient includes an elongate, flexible cloth fabric but having strap looping means mounted on its central segment. An adjoined pair of elongate, interruptable adhering means are mounted on one side of each of the extended length of the belt segment. A ring-like closure member forms each of the free ends of the extended lengths and a depending pair of restraining straps are secured at their one free end to the front side central belt segment and are of length to make a looping engagement with the intermediate segments of the central belt fore ends.

One anchoring strap is provided for each fastening strap, with each such anchoring strap being adapted to cooperate with its respective fastening strap, with the length of the anchoring strap passing through the attachment means of the fastening strap. Each of the anchoring straps terminate in mateable elements which form an anchor strap joinder and release means.

## Description

The present invention relates to a limb restraint device including a padded flexible member, an attached cloth means forming dual symmetrical loops and aligned pairs of complemental hooks- locking segments located on the opposing planar surface at the opposing ends of the fabric member. A separable anchoring strap engages slidingly with the symmetrical loops, providing overall controlled play of the limb.

The present invention also discloses safety belts for a chair-bound patient to preclude slipping down and out of the chair. The unique belt is cooperatively associated with the safety vest proper.

A wide range of patients must be protected against unsafe and unhealthful movement -- principally falling out of body supports such as a bed, tables and chairs, or by moving their body portions, and thereby rupturing sutures, or otherwise causing further injury to already impaired body portions. Without being secured in such supports by health care personnel, such patients are likely to cause serious injury to themselves. Such patients include comparative invalids, as well as those who have sufficient consciousness and strength to attempt to disengage such restraints; or to engage in substantial movement, but who are also subject to sufficient aggression, disorientation, or other debilitating condition, that disengaging their own restraints would likely result in injury to them. However, since the subject being restrained is a patient who is suffering from a medical disability, such restraint must be comfortable and not overly confining in use to be acceptable.

In sum, despite their long history of use, and the variety of forms offered, serous mishaps do occur to agitated patients, even when currently available vest or limb restraints are employed.

It is a principal object of this invention to provide a limb restraint device adapted to limit excess limb flexing including padded flexible fabric member and cloth adapted for double looping and cooperation with an adjustable anchoring means of the prior art.

A still further object of the invention is to provide a belt-like harness for the lower patient torso, which cooperates with the U.S. Patent 4,832,053 and W089/09581 vest restraint and so precludes a vested patient, who is also a chair-bound, from wriggling down and out of the supporting chair even while his upper torso is adequately vested.

Other objects and advantages of the present invention will become apparent from the following specification and from the drawings and the claims.

According to the invention, in a first aspect there is provided; a safety harness for enclosing the lower torso of a seated patient comprising an elongate flexible fabric belt having a central segment and two pairs of strap loop means mounted spaced apart on the central segment; a closely adjacent pair of elongate complemental adhering means mounted on one side of the central segment, the paired adhering segments being located intermediate the looping means on the free end of the elongate strap free end; a ring-like closure member secured to each strap free end; a depending pair of fabric restraining straps, being each secured at one longitudinal end to the belt front side and being of sufficient length to comfortably encircle the lower patient torso; and a pair of flexible cross members, each attached proximal to the free ends of the opposing restraining straps, and thereby limit the strap tendency to diverge unduly when mounted about the patient's buttocks.

In the other major aspect of the invention, there is provided a limb restraint device suited for readily interruptable contact with a limb comprising a first flexible fabric member having some integral padding of a rectangular planar configuration; a flexible cloth, ribbon-like means presenting a closed loop fastened diametrically to the opposing margins of the fabric member, thus forming two hemispherical loops adapted for looping engagement with a linear strap; a first pair of complemental pile and hook-locking segments mounted on one planar surface proximal to one longitudinal end of the fabric member; a second pair of complemental, pile and hook-locking segments similarly mounted on the opposing surface and proximal to the other longitudinal end of the fabric member; the complemental segments of both pairs being arrayed to overlap and have the first pair make secure contact with the opposing first pair of adhering segments; and a separable anchoring strap adapted to cooperate with the two loops along the strap mid-length by passing through the loops, and terminating in an anchoring strap joinder and release means; the strap being of a length sufficient for the engaged strap to also be looped around a remote support post, which is well spaced apart from the limb (wrist) restraint device itself, whereby the overall flexing play of the thusly restrained limb is controlled by the associated anchoring strap.

The invention will be further described in connection with the drawings in which
Fig. 1 is a plan view of the anchoring strap component of the invention, functionally identical to the anchoring strap depicted in Fig. 4 of U.S. Patent 4,832,053 (W089/09581), but modified only slightly as to one of the terminal mating elements at its longitudinal end as to ring configuration, with such anchoring strap being securable to remote stationary posts in a ready manner, (see Fig. 2, 3, and 4, next to be described);
Fig. 2 is a perspective view of the chest side of a safety belt adapted for a patient's lower torso useful in cooperation with the safety vest described in the afore tested U.S. Patent '053, and showing a restrained (partial) patient torso in phantom;
Fig. 3 is an elevational view of the reverse side of the safety belt of Fig. 5, as it protectively engages a seated patient's lower rear torso, and also depicts an offset pair of spaced-apart, vertical harnesses which embrace the patient's buttocks;
Fig. 4 is an elevational view of the safety belt device (stomach side) but with the extending out of the now unfastened free ends, so to depict the underlying complemental set of fastener segments located on each of the respective extended fastener straps, which engage securely during the looping back, leaving the ring-like, longitudinal ends adapted for anchor strap coupling;
Fig. 5 is an elevational view of one surface of a device used for patient wrist restraint, depicting a centrally mounted loop-like fastening means and depicting one of the end sets of adjacent, complemental fastener pads;
Fig. 6 is an elevational view of the opposing planar surface of the wrist restraint device of Fig. 5 depicting the other end set of complemental fastener pads, being adapted to use with the above described restraint and safety belt; and
Fig. 7 is a perspective view of one of the wrist restraint devices in use cradling a patient forearm, and with loop-like means now operatively associated with the intermediate length of an anchoring strap of Fig. 1.
Fig. 8 is a plan view of an alternate embodiment of an anchoring strap, especially adapted for cooperating with the patient wrist restraint device of Fig. 5, in place of the standard anchoring strap 100 of Figs. 1 and 7;
Fig. 9 is a top elevational view of the anchor strap of Fig. 8 functionally entwined with a limb restraint device of Fig. 8 prior to actual limb engagement; and
Fig. 10 is a perspective view of how the alternate anchor strap of Fig. 8, cooperating usefully with a human wrist being restrained by the limb restraint, thereby depicting how restrained limbs can be safely tethered to a support chair in which a patient has been seated.

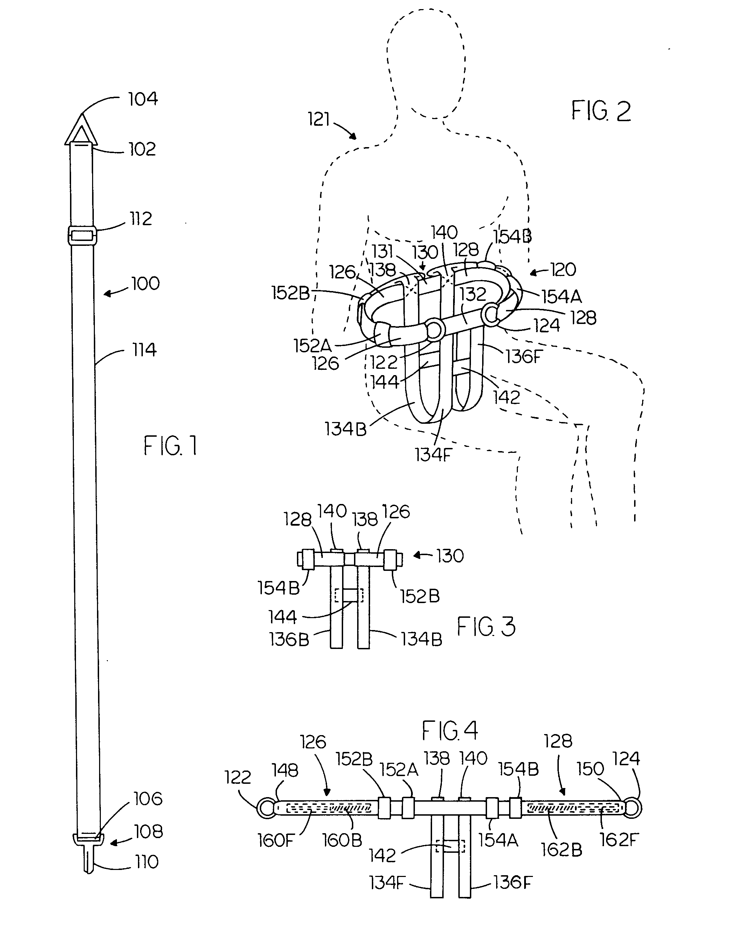

In Fig. 1, is a top plan view of the anchoring strap component 100, laid out in its full extension, as it would appear before its use in the present invention. At its one longitudinal end 102, the anchor strap terminates in a rigid, conveniently triangular attachment means, such as a metal (or formed plastic) ring 104. This ring can be secured similarly to rings 27, 28, employed on the free ends of the fastener straps 16, 17 of Fig. 1 of W089/09581.

The other longitudinal end of anchor strap 100 is looped through a transversely configured, rigid slot 106 of fastener 108. Slot 106 is integral with the conventional snap spring fastener 108, typically one with a partial hook-shaped rigid end and a cooperating flexible metal (or plastic) snap strip 110. Snap 110 is biased to be normally closed within the inner tip (not seen) of the hooked end. Strip 110 is adapted to be manually depressed so as to permit instant release of any ensnared ring, like closure means 104 of Fig. 1.

Ring means 104 of anchor strap 100 (Fig. 1) is the one with which the other complemental snappable joinder means 108 will cooperate, after the anchoring strap 100 is properly looped through a safety vest fastener strap rings. The elongate free fabric portion of anchor strap 100, which is located intermediate ring 104 and snap fastener 110 (not seen), is held by a conventional double-slot, rigid buckle 112. The slidable buckle 112 tracks itself along on the free fabric length 114 of the anchor strap 100 proper. In this manner, the overall length of anchor strap 100 is substantially variable, but is also still a presetable one, so as to tightly straddle the distance between the vest attachment point (its fastener rings), and the particular type of remote stationary post(s), to be employed with the vest while in use, usually bed railings.

As for the juxtapositions of the Velcro fastener, as to its complemental components, the adhesive pile component may be more conveniently mounted proximal to the tied ends of the fastening straps, while the multiple hook pads are mounted proximal to the free ends of the fastener straps. Either arrangement is operable, but that just described is preferred for ease of manipulation of fastening straps.

An ancillary device that can be usefully employed with the just described safety vest is the belt restraint means of Figs. 2 to 4. In the perspective view of Fig. 2, such a safety belt 120 is depicted, as it would appear mounted on the lower torso of a seated patient 121, who may also be wearing a safety vest, as well. If so, the free ringed ends 122, 124 of dual horizontal belting straps 126 and 128 of belt 120 would be threaded through the lower loops 33C and 33D on the prior art vest of W089/09581 before they are doubled back to engage belt rear midsection 131 for secure fastening, as will be described.

The front midsection 132 of belt 120 presents a spaced-apart, pair of vertically oriented restraining straps 134F and 136F, which then are passed under the patient crotch and buttocks, to circle back so as to be securely attached via terminal end loops to the backside 131 of the belt, i.e., to double-looped horizontal belt mid-segment 140.

To maintain a suitable positioning athwart the patient's lower torso, at least two connecting segments 142 and 144 span the gap between the frontal and backside runs of the parallel vertical straps 134F/B and 136F/B. The rear segment of belt 120, also has upper connecting segment 131, which is flanked laterally by the permanently looped ends of vertical straps 134B, 136B. These vertical rear loops 138 and 140 (also Fig. 4) are permanently secured to either end of the horizontal mid-segment 131, and serve as the doubling back terminal for the free ends 148, 150 of belt forming straps 126 and 122, as shown better in Fig. 4.

Each of the free ends of belt forming straps are provided with the ring-like element 122, 124, respectively, which will each engage the snap-clip ends 110 of the cooperating anchoring straps 110 (Fig. 1), which were described above. Proximal to the lateral sides of opposing straps 126/128 are mounted vertically-oriented, flexible pairs of loops 152A/B, and 154A/B, respectively, through which the belt strap free longitudinal ends 148/150 are passed, before their snap-clip engagement with associated anchoring straps.

In the reverse-side view of Fig. 3, the mode of fixed rear side attachment of buttocks-encircling vertical straps (136B/134B), to the double back runs of the horizontal belt loops 124/126 is shown. Also, near the lateral sides of horizontal belt segment are vertically oriented, outer soft loops 152B and 156B, which also serve to contain the closed free ends 148/150 of belt girth straps 126/128, when a patient of large lower girth is being protected.

The elevational view of Fig. 4 is of the safety belt front side, but now depicted with the horizontal belt straps 126, 128 disengaged from their functional, Velcro-type retention. This reveals the substantial length, of the underlying pads of complemental fastening material, fixedly mounted on the inside of the horizontal belt. Preferably, the adhesive pile segment is on the inner belt portion 160A/162B, and the hook-locking segment 160F/162F, is disposed on the outer strap portion.

Both the restraining vest and cooperating safety belt of the present invention provide a marked improvement over earlier known garments. They provide for secure restraining means, which cannot be released by the restless patient, since the sliding buckles, like 112 and the snappable fastener, like 110 on anchoring strap 100, are located distally from a patient; yet, it is these components that are comparatively inexpensive to fabricate, requiring no unusual hardware. They also involves no integral component which can become hazardous to the patient.

Safety belt 120 of Figs. 2 to 4, can be employed in conjunction with the safety vest of copending Ser. No. 590,496 (EPO Reference Number PCT International Publication #W089/09581 dated 19 October 1989), so as to provide added security with a seated patient. Firstly, left end ring 122 of the belt passes through one lower loop 25 on the vest front panel 21. Be sure that both front loops (152A/154A) on the belt pass through the lower loops on the vest. Repeat the step, threading the other belt ring 124 through the opposing front loop 26 on the vest. Again, both the belt strap free ends should slide through the vest loop. Later, when resorting to the anchoring straps (Fig. 1), one will need to couple the belt rings 122/124, with the strap rings like 28.

The dual strand, webbed material, elongate crotch strap 134F/136F should be passed between the patient's legs and doubled back to the rear panels on the safety vest. To connect this webbed section to the side belt strap, thread each belt ring, 122/124, through one loop (154B) on the terminal end of the webbed section. One one end, double back the strap 128 free end 150, matching the Velcro pad in the strap center, and then thread the end ring 124 of the belt through the dual side loops 154F/159B.

Repeat these steps for the other belt strap 126, threading, doubling back and matching the Velcro pads, finally threading end ring 122 through paired loops 152A/152B. Now, the integrated safety vest and belt are properly enclosed about the patient who has been secured, as needed, in a wheelchair, or safety chair. Safety belt 120 is not usually employed with a bed-ridden patient.

Most impressively, it handily permits necessary readjustments of the patient's body, without risking an injurious fall from the supporting bed, or any contortions by an agitated patient that could convert the vest edges into a strangulation ligature, as has been discussed.

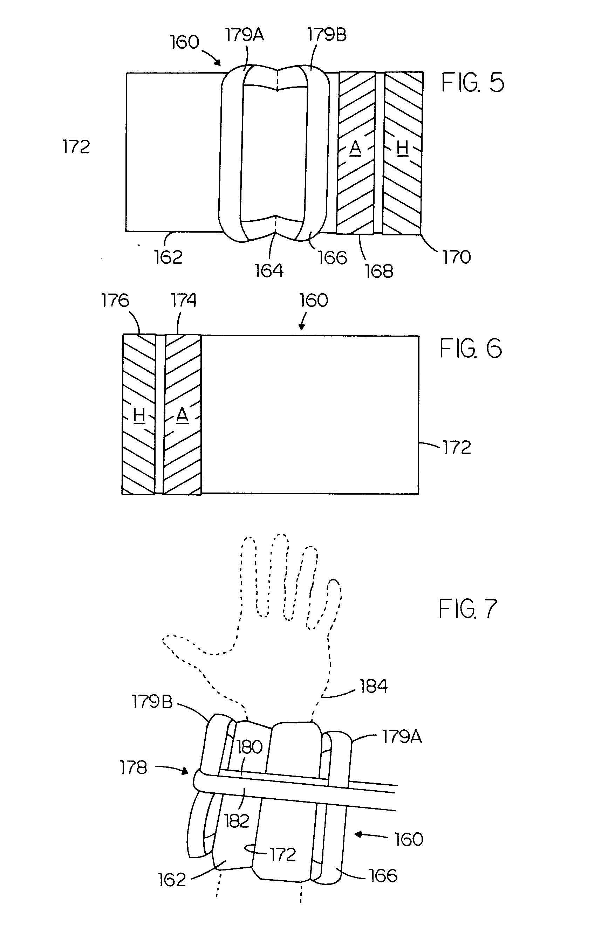

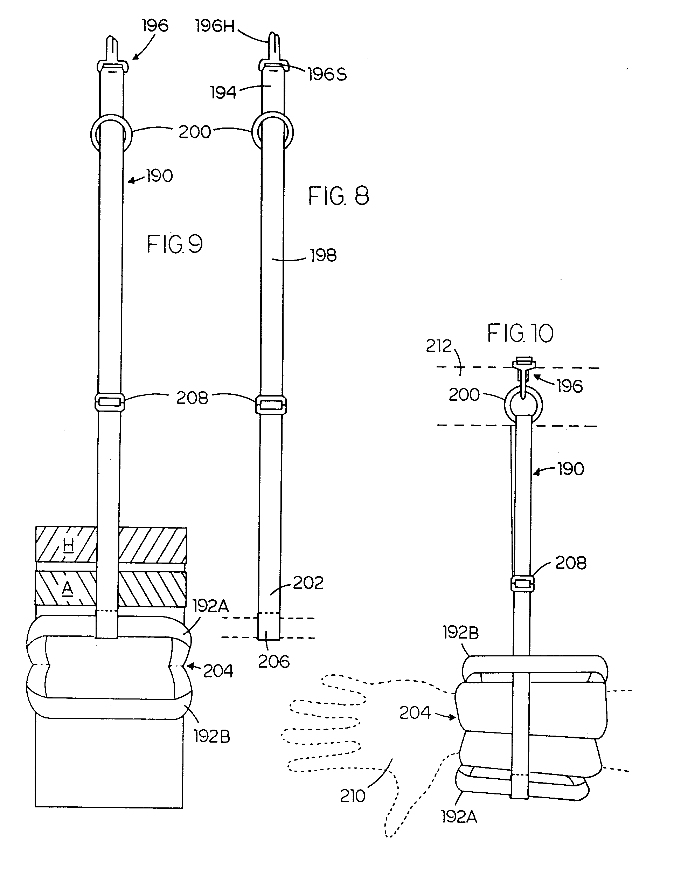

The ancillary device of a wrist-cuffing means of Figs. 5 to 7, is provided for those agitated patients needing limb protection, as well. The cuffing means has a fully padded, flexible fabric base member, which is mateable with complimentary pairs of Velcro adhering pads and a loop-like fastening means. As will be described, the wrist-engaging cuffing means is thusly coupled to a separate anchoring strap (Fig. 1or Fig. 8), which may be secured to the bed-frame, as seen in Figs. 5 and 6 of my U. S. Patent 4,832,053.

The wrist restraint device 160 of Fig. 5 is comprised essentially of a flexible, but strong, rectangular, integrally-padded, woven cloth 172, (such as a core composed of foam-shaped elastomer), on which surface are mounted certain operating elements, to be described. Secured centrally on one planar side (facing), in the central portion 164, is a fabricated endless cloth fastening means 166 (present in a manner such as to double loops). As mounted, it describes a symmetrical closed loop configuration in its relaxed state, and is bonded linearly to the underlying padded cloth 172 at its upper and lower margins along one central transverse length. On the right side of closed loop 166 is an adjacent pair of complemental securing segments, which serve for locking, as in the aforedescribed restraint vest and belt safety harness.

In one embodiment, inner pad 168 can be the adhering Velcro pad type, while outer pad 170 is then of the complemental multiple-hook type. The square-cut, same side, opposing longitudinal end 172 of protective device 160 is of unmodified padded, woven cloth running from the center attachment line 164 of loop means 166.

On the opposing planar surface of restraint device 1600, shown in Fig. 6, note it has been modified to include at its other longitudinal end 172 only a substantially identical, adjacent pair of complemental securing segments, 174 and 176, located and mounted transversely of the padded cloth, and located very proximal to the left hand side, of longitudinal end segment 172. Consistent with the nature of the inner side pad set (1688/170), inner pad 174 is of the adhesive pad type, since outer pad 170 of Fig. 5 is of the multiple hook type, so they will adhere firmly upon making contact. Likewise, since outer pad 176 (Fig. 6) is also of the multiple hook type, then inner pad 168 (Fig. 5) is of the adhesive pad type, for manifest intermittent closure reasons.

Thusly, the complemental segments of each cloth longitudinal end pair are arrayed so that when one cloth restraint longitudinal end 172 is folded about a limb, and it then overlaps the other squared cloth end 174, then the adjacent adhering segments of the one securing pair will make a secure, but still interruptable, binding contact with the securing segments of the other pair while in use.

So, once a wrist is cradled within the overlap- pable and adhering end pads of wrist restraint 160, then the adjacent padded ends 172/174 of Fig. 5 are enfolded cradle-like around the cradled wrist, are drawn in, and then engaged with anchoring strap 100 (Fig. 1), the practical result is as is now depicted in Fig. 7.

The thusly cradled cuffed wrist 184 is indirectly linked to an anchoring strap 178, which has its one inner segment length 180 slipped under and around both of flexible loops 179A and 178B, and its doubled-back segment 182 running backwards to the hook end 110 (not seen) of the anchor strap 100 itself (Fig. 1). Anchor strap 178 is secured at the other closed end (not seen), just as are the discrete anchoring straps 100 that are employed with the prior art restraint vest and the safety belt device of this invention. The taught dual sets of Velcro-type end pads will provide more stability to each wrist cuff and its associated anchoring strap. Optionally, an adjacent adhering pair can be located on the one cloth surface and an adjacent hooking pair on the other opposing cloth surface.

To employ the wrist-cuffing device, first wrap the patient's wrist 184 in the flexible, open cloth segment 174 of Fig. 6, insuring physical comfort and safety.

Confirm that the complemental Velcro pads (168/176 and 170/174) do make adhering contact on the underneath side of the wrist. Next, enfold the central loops 179A/179B about the limb-engaged padded restraint. Then take the snap-hook end 108 of strap 100 (Fig. 1), and pass it through both loops 179A/179B on the fastening means 166. Reattach the anchor strap free end 104 about the bed frame, as earlier described. Preadjusting of the anchoring strap length may be in order to confirm proper strap tension or leeway for wrist motion.

A modified anchor strap 190 of Fig. 8 is seen laid out in its full extension before it is to be coupled with either one of the hemispherical loops 192A, 192B, of the limb restraint of Fig. 9. At the upper one strap longitudinal end 194 are seen a free-running snap fastener assembly 196, composed of its base slotted element 196S, which can travel as mounted on the width of fabric strap 198, and its linked outwardly projecting snap hook means 196H. Also, running-free along strap 190 is a formed plastic ring 200, adapted to couple with the snap hook means of fastener assembly 196, at the proper location, as will be described.

At the other longitudinal end 202 of anchor strap 190 is seen the free fabric end of the strap, before it is entwined with one of the hemiloops (192) of restraint device 204; it was previously doubled back on itself and is secured, as by sewing, to the proximal segment of the same longitudinal end, thus presenting a permanently engaged end loop 206.

When the strap nonfastener end 202 is thusly closed, such end loop is threaded, deployed and associated with restraint device 204; then it presents itself as seen in the perspective view of Fig. 9, just prior to limb envelopment. This will permit each wrist and/or leg restraint device to have its own tieless anchor strap, adapted to tether that specific limb to a chair arm rest or chair leg, while gaining the safety advantage of containing erratic and extreme limb movements by an agitated patient. Strap 190 needs to be of an intermediate length sufficient to be looped about a support post. The overall length of anchor strap 190 is purposefully variable, as is that of prior anchor strap 100 (Fig. 4), simply by including along its intermediate length, the adjustable buckle 20, which is identical to the like buckle 112 of the anchor strap of Fig. 4.

The limb restraint 204 and cooperating anchor strap 190 are shown in functional engagement with a patient wrist 210 (Fig. 10) while being remotely tethered to a fixed post 212, which is either a chair arm or bed frame side rail. Anchor strap 190 has been somewhat foreshortened via buckle 208 to provide the tolerable amount of limb play. As seen in Fig. 7, the restraint 204 has been wrapped about the wrist, while the entwined end 206 of strap 190 runs back under opposing loop 192B and thence to fixed railing 212. Quick release of the tether is effected at the railing end and threading back the anchor strap back under loop 192B to open the padded wings to prompt release.

Per this invention, a separable anchoring strap is provided adapted to cooperate with the hemispherical loops of said ribbon-like means along the intermediate length of said anchoring strap, by threading one looped longitudinal end of said anchor strap so it is slidingly entwined with at least one of the generally hemispherical loops of said ribbon-like means; and by providing the other longitudinal end of said anchor strap with a pair of free-running mateable elements, which form an anchor strap joinder and release means. The strap itself being of an intermediate length sufficient for it also to be looped around a support post, which is variably spaced apart from the wrist restraint device itself; whereby overall flexing play of the restrained limb is controlled by the associated anchor strap running through one of the hemispherical loops of the ribbon-like means and the anchor strap release means is not practically accessible to the restrained patient.

The present invention has been described with reference to a presently preferred embodiment thereof. Such embodiment should not considered a limitation of the scope of the present invention. The scope of the present invention is better ascertained by reference to the following claims.

## Claims

1. A restraint device adapted for interruptable contact with a restrained limb and for concurrent anchoring to limit exaggerated limb flexing, comprising:
(a) a flexible fabric member having integral padding substantially throughout and being of a generally rectangular configuration;
(b) a flexible cloth ribbon-like means presenting a closed loop and being fastened diametrically to the opposing upper and lower margins of the fabric member along one central transverse dimension thereof, such ribbon-like means thus forming two symmetrical hemispherical loops adapted for double looping engagement with a linear strap;
(c) a first pair of complemental pile and hook-locking segments mounted adjacently and transversely on one surface of said fabric member and being proximal to one longitudinal end of said fabric member;
(d) a second pair of complemental pile and hook-locking segments mounted adjacently and transversely on the opposing surface of said fabric member and being proximal to the other longitudinal end of said fabric member;
(e) the complemental segments of both pairs being arrayed so that when the one longitudinal end of the padded member is folded about a limb-like member, and acts to overlap the other long end, that the pile segment of the first pair, will make secure contact with the hook-locking pad segment of the second pair, while the hook-locking segment of the first pair will make secure contact with the pile segment of the second pair, and
(f) a separable anchoring strap adapted to cooperate with the two hemispherical loops of said ribbon-like means along the intermediate length of said anchoring strap by passing through said loops while they are disposed in their limb restraining mode, with the opposing ends of said anchoring strap itself terminating in mateable elements which form an anchor strap joinder and release means, and being of an intermediate length sufficient for such engaged strap to also be looped around a support post which is spaced apart from the wrist restraint device, whereby the overall flexing play of the restrained limb is controlled by the associated anchoring strap running through the double loops of the ribbon-like cloth means.

2. The restraint device of Claim 1 in which the mateable elements of the anchoring strap comprises a ring-like closure means secured at its one free end and a manually-activated hooking means disposed at the other strap free end.

3. The restraint device of Claim 1 or 2 in which the complemental adhering segments of both pairs are rearranged so that when the one fabric member longitudinal end is folded about to overlap the other long end of the fabric member, that an first pair of adjacent pile segments will then make secure contact with the opposing second pair of adjacent hook-locking segments that are disposed oppositely at the other longitudinal end.

4. The safety harness of any of Claims 1 to 3, wherein the complemental pad means mounted upon each of the extended lengths of the horizontal belt portion further comprises on each opposing length, a locking hook pad segment which is located most proximal to the free end of the belt and an adjacent adhesive pad segment which is located intermediate of the adhering pad segment and the first pair of strap looping means located along that extended length of the belt portion.

5. A restraint device adapted for interruptable contact with a restrained limb and for concurrent anchoring to limit exaggerated limb flexing, comprising:
(a) a flexible fabric member having integral padding substantially throughout and being of a generally rectangular configuration;
(b) a flexible cloth ribbon-like means presenting a closed circle and being fastened diametrically to the opposing upper and lower margins of said fabric member along one central transverse dimension thereof, such ribbon-like means thus forming two symmetrical hemispherical loops adapted for double looping engagement with a linear strap;
(c) a first pair of complemental pile and hook-locking segments mounted adjacently and transversely on the surface of said fabric member and being proximal to one longitudinal end of said fabric member;
(d) a second pair of complemental pile and hook-locking segments mounted adjacently and transversely on the opposing surface of said fabric member and being proximal to the other longitudinal end of said fabric member;
(e) the complemental segments of both pairs being arrayed so that when the one longitudinal end of the padded member is folded about a limb-like member, and acts to overlaps the other long end, that the pile segment of the first pair, will make secure contact with the hook-locking pad segment of the second pair, while the hook-locking segment of the first pair will make secure contact with the pile segment of the second pair, and
(f) a separable anchor strap adapted to cooperate with the hemispherical loops of said ribbon-like means along the intermediate length of said anchoring strap by threading one longitudinal end of said anchor strap so it is slidingly entwined with at least one of the generally hemispherical loops; and providing the other longitudinal end of said anchor strap with a pair of free-running mateable elements, which form an anchor strap joinder and release means, said strap being of an intermediate length sufficient for it to be looped around a support post, which is variably spaced apart from the wrist restraint device itself, whereby overall flexing play of the restrained limb is controlled by the associated anchor strap running through one of the hemispherical loops of the ribbon-like means and the anchor strap release means is not practically accessible to the restrained patient.

6. A safety harness for reliably enclosing the lower torso of a seated patient inclined to restlessness, which comprises:
(a) an elongate, flexible fabric belt having a free fabric central segment and two pairs of strap looping means being mounted symmetrically apart on said central segment, with all the loops located on the same strap side, with the inner loops also spaced-apart a distance approximating the frontal width of a human torso;
(b) a closely adjacent pair of elongate, interruptable, complemental adhering means adjacently mounted on the one side of the extended lengths of the central belt segment, such paired segments being located between one pair of looping means on one strap side and the free end of said elongate strap on the same side;
(c) a separate ring-like closure member secured to each of the free ends of said elongate belt;
(d) a depending pair of fabric restraining straps, spaced-apart, and secured at one longitudinal end to the front side, central belt segment of said elongate belt, being of sufficient length to comfortably encircle the lower patient torso, and being adapted at its other longitudinal end to make an interruptable looping engagement with each of the intermediate segments of the main belt extended free ends; and
(e) a pair of flexible cross members, each being located and attached proximal to each of the free ends of each of the restraining straps, being adapted to limit the tendency of each parallel restraining strap to diverge excessively when mounted about the buttocks of a patient.

7. The safety harness of Claim 6 wherein the complemental pad means mounted upon each of the extended lengths of the horizontal belt portion further comprises on each opposing length, a locking hook pad segment which is located most proximal to the free end of the belt and an adjacent adhesive pad segment which is located intermediate of the adhering pad segment and the first pair of strap looping means located along that extended length of the belt portion.
